# EUROPEAN PATENT APPLICATION

(11) **EP 4 681 748 A1**
(43) Date of publication of application: **21.01.2026**
(21) Application number: 24189584.6
(22) Date of filing: 18.07.2024
(51) Int. Cl.: A61M 5/315

(54) **SYRINGE PLUNGER ROD WITH DISTAL END BARREL CONTACT**

(71) Applicant: Becton, Dickinson and Company, Franklin Lakes, NJ 07417 (US)
(72) Inventor: PILOY, Jefferson, 38000 GRENOBLE (FR); LAVIGNE, Ferdinand, 38170 Seyssinet-Pariset (FR); BOURGEOIS, Réjane, 38000 GRENOBLE (FR)
(74) Representative: Germain Maureau

(57) **Abstract**

A syringe is provided comprising a syringe barrel having a chamber configured for containing a fluid therein, and a plunger assembly axially movable within the chamber of the syringe barrel from a retracted position to an advanced position. The plunger assembly includes a plunger rod having a plunger rod proximal end and a plunger rod distal end, the plunger rod comprising a distal end head positioned at the plunger rod distal end. The plunger assembly also includes a stopper secured to the plunger rod distal end, the stopper having a proximal end and a distal end. With the plunger rod and stopper of the plunger assembly assembled, the distal end head is configured to abut the stopper without being mechanically coupled thereto, so that the plunger rod may be separated from the stopper and removed from the syringe barrel by applying only a rearward axial force on the plunger rod.

## Description

### BACKGROUND OF THE INVENTION

### Field of the Invention

The present disclosure relates generally to a plunger assembly for use with a syringe and, more particularly, to a plunger assembly that includes a plunger rod having a distal end head that provides for a distal advancement of the stopper within the syringe barrel without attaching to the stopper, with the distal end head maintaining barrel contact with the syringe barrel.

### Description of Related Art

Medical injection devices, such as syringes, are used in a variety of environments for administering liquids (e.g., medications or drugs) to a patient. Many syringes are provided as prefilled syringes, which provide the convenience of rapidly delivering the liquid therein to a patient without the need to first aspirate the medication from another container and meter its volume. For dispensing fluids, the prefilled syringe will typically include a syringe barrel with a plunger assembly inserted through an open proximal end of the barrel and an opening at the opposite distal end adapted to receive a needle therein by which a fluid is injected into the patient. The plunger assembly typically includes an elongated plunger rod extending out of the barrel, and a plunger head or stopper disposed at the distal end of the plunger rod. The stopper is typically made of elastomeric material and is adapted to ensure the container closure integrity (CCI) of a syringe when the stopper is inserted into the syringe.

Existing stopper designs include a stopper body having a tail portion disposed at its proximal end adapted for attachment to the distal end of the plunger rod, and a head portion disposed at its distal end adapted to interfit with the barrel of the syringe. The tail portion of the stopper may include a cavity formed therein configured to receive a distal head of the plunger rod, so as to engage the plunger rod with the stopper. According to some embodiments, each of the cavity and the plunger rod head may be configured to provide a threaded engagement therebetween to secure the plunger rod to the stopper.

With a prefilled syringe, it is common for the syringe to be considered a "single-use syringe" - with the entirety of the syringe (syringe barrel and plunger rod) being discarded into a hazardous waste bin after use. However, sustainability and financial considerations have been driving end-users and manufacturers toward the re-use or recycling of certain components of the syringe where possible. Specifically, the plunger rod is a candidate for re-use or recycling, as the plunger rod is typically a plastic component capable of such re-use or recycling.

Despite this identifying of the plunger rod as a candidate for re-use or recycling, it is recognized that certain obstacles are present that might prevent separation/disconnection of the plunger rod from other syringe components so as to provide for the re-use or recycling thereof. For example, the typical plunger rod and stopper construction requires that the plunger rod be unscrewed from the stopper in order to separate the components. This unscrewing of the plunger rod from the stopper may be difficult for an end-user for a number of reasons, including the high amount of torque that must be applied to unscrew the plunger rod from the stopper and/or the lack of friction between the stopper and the syringe barrel that may allow the stopper to rotate along with the plunger rod when torque is applied thereto.

Accordingly, a need exists in the art for a plunger rod design that provides for separation of the plunger rod from the stopper and removal of the plunger rod from the syringe barrel following use of the prefilled syringe. The design would ensure that use of the syringe is not impacted, but would enable post-injection removal of the plunger rod from the stopper and the syringe barrel without the need for a high torque being applied to unscrew the plunger rod from the stopper.

### SUMMARY OF THE INVENTION

Provided herein is a syringe comprising a syringe barrel having a barrel proximal end and a barrel distal end and defining a chamber configured for containing a fluid therein, and a plunger assembly axially movable within the chamber of the syringe barrel from a retracted position to an advanced position. The plunger assembly includes a plunger rod having a plunger rod proximal end and a plunger rod distal end, the plunger rod comprising a distal end head positioned at the plunger rod distal end. The plunger assembly also includes a stopper secured to the plunger rod distal end, the stopper having a proximal end and a distal end. With the plunger rod and stopper of the plunger assembly assembled, the distal end head is configured to abut the stopper without being mechanically coupled thereto, so that the plunger rod may be separated from the stopper and removed from the syringe barrel by applying only a rearward axial force on the plunger rod.

In some embodiments, the stopper comprises an inner cavity formed therein at the proximal end, with an annular shoulder at the proximal end of the stopper defining an opening to the inner cavity.

In some embodiments, the distal end head comprises a disk-shaped end member having a flat distal-facing surface.

In some embodiments, the disk-shaped end member has an outer diameter that is greater than an inner diameter of the cavity and approximately equal to an inner diameter of the syringe barrel, with the disk-shaped end member thus in contact with an inner surface of the syringe barrel.

In some embodiments, with the plunger rod and stopper of the plunger assembly assembled, the disk-shaped end member abuts the annular shoulder of the stopper.

In some embodiments, the disk-shaped end member has a thickness that provides for deforming of the disk-shaped end member as the plunger assembly is advanced distally into the syringe barrel during an injection.

In some embodiments, the thickness of the disk-shaped end member is between 0.01 and 0. 5 mm.

In some embodiments, the syringe includes a gasket positioned about the plunger rod adjacent the disk-shaped end member.

In some embodiments, the is gasket is formed of an elastomeric material.

In some embodiments, with the gasket positioned about the plunger rod, the gasket the has an outer diameter that is approximately equal to an inner diameter of the syringe barrel, with the gasket thus in contact with an inner surface of the syringe barrel.

In some embodiments, the gasket is in interference with the inner surface of the syringe barrel by 0 to 0.5 mm.

In some embodiments, the distal end head comprises a multi-legged end member, with the multi-legged end member including a central hub having a flat distal-facing surface and a plurality of flexible legs joined to the central hub and spaced circumferentially thereabout, with each of the plurality of flexible legs comprising a first end that is joined to the central hub and a free second end, with each of the plurality of flexible legs having a thin, elongated profile that allows the leg to bend and flex responsive to a pressure applied thereto, wherein the plurality of flexible legs are configured to extend back proximally from the central hub and flare radially outward therefrom.

In some embodiments, each of the plurality of flexible legs comprises a curved or arc-shaped leg.

In some embodiments, the central hub has an outer diameter that is less than an inner diameter of the cavity such that, with the plunger rod and stopper of the plunger assembly assembled, the central hub is inserted into the cavity.

In some embodiments, a circumferential arrangement of the plurality of flexible legs, at the second ends thereof, has an outer diameter that is approximately equal to an inner diameter of the syringe barrel, with the second ends of the plurality of flexible legs thus in contact with an inner surface of the syringe barrel.

In some embodiments, with the plunger rod and stopper of the plunger assembly assembled and the central hub inserted into the cavity, the plurality of flexible legs bend to enable the insertion.

In some embodiments, the plurality of flexible legs comprises four legs.

In some embodiments, the distal end head maintains contact with an inner surface of the syringe barrel, without increasing a gliding force of the plunger assembly during a distal advancement thereof within the syringe barrel.

In some embodiments, the distal end head is formed integrally with a main body of the plunger rod.

In some embodiments, the distal end head is coupled to a main body of the plunger rod.

In some embodiments, the syringe comprises a pre-filled syringe.

### BRIEF DESCRIPTION OF THE DRAWINGS

FIG. 1 is a perspective view of a syringe, with which embodiments of the disclosure may be implemented;
FIG. 2 is an exploded view of the syringe of FIG. 1;
FIG. 3 is a side view of the stopper included in the syringe of FIG. 1, according to a non-limiting embodiment described herein;
FIG. 4 is a perspective view of the distal end head of the plunger rod included in the syringe of FIG. 1, according to a non-limiting embodiment described herein;
FIG. 5 is a side cross-sectional view taken along line A-A of FIG. 1, showing the distal end head of FIG. 4 engaged with the stopper;
FIG. 6 is a perspective view of the distal end head of the plunger rod included in the syringe of FIG. 1, according to another non-limiting embodiment described herein;
FIG. 7 is a side cross-sectional view taken along line A-A of FIG. 1, showing the distal end head of FIG. 6 engaged with the stopper;
FIG. 8 is a side view of the distal end head of the plunger rod included in the syringe of FIG. 1, according to another non-limiting embodiment described herein; and
FIG. 9 is a side cross-sectional view taken along line A-A of FIG. 1, showing the distal end head of FIG. 8 engaged with the stopper.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the present disclosure, the distal end of a component or of a device means the end furthest away from the hand of the user and the proximal end means the end closest to the hand of the user, when the component or device is in the use position. Thus, with a syringe for example, the distal end is the end thereof that includes a needle (or luer connection), while the proximal end is where the user engages the plunger (i.e., the plunger thumb press). Similarly, in this application, the terms "in the distal direction" and "distally" mean in the direction toward the distal tip of the syringe, and the terms "in the proximal direction" and "proximally" mean in the direction opposite the direction of the distal tip of the syringe (i.e., in the direction of the plunger thumb press).

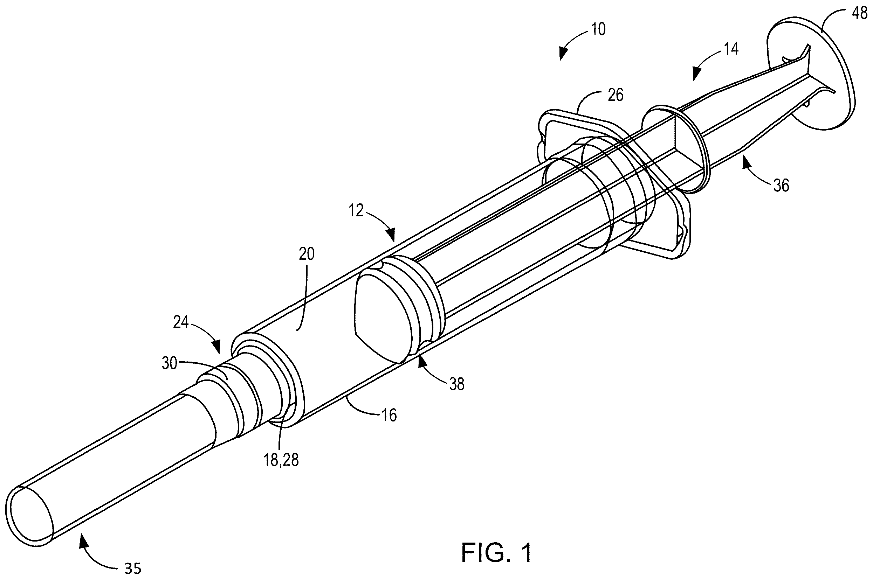

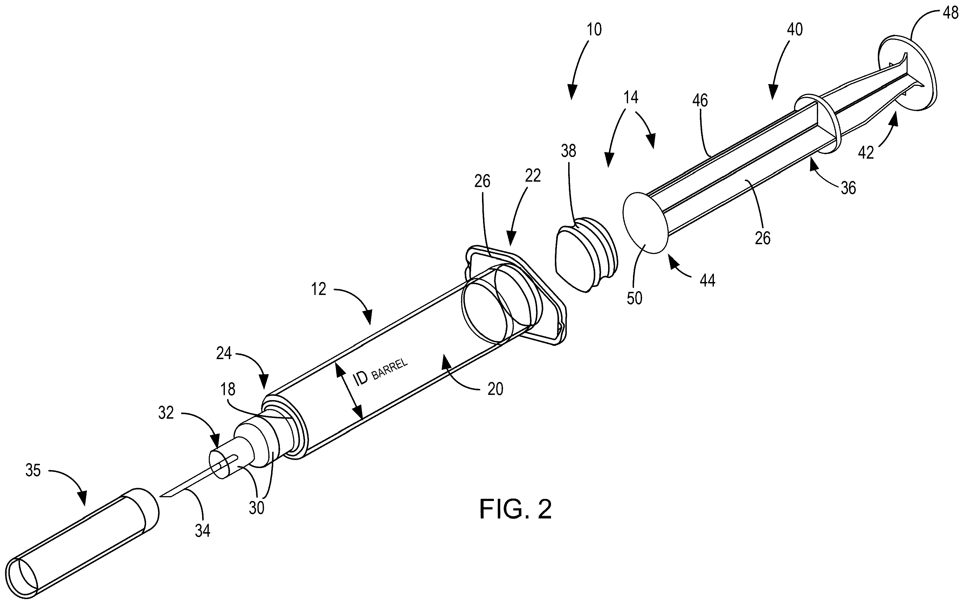

Referring to FIGS. 1 and 2, shown is a non-limiting embodiment of a syringe 10 with which aspects or embodiments of the disclosure may be implemented. According to some aspects of the disclosure, the syringe 10 may be provided as a prefilled syringe, which provides the convenience of rapidly delivering the liquid therein to a patient without the need to first aspirate the medication from another container and meter its volume.

As shown in FIGS. 1 and 2, the syringe 10 generally includes a syringe barrel 12 and a plunger assembly 14. The plunger assembly 14 is movable within the syringe barrel 12 along a longitudinal axis to an advanced position to facilitate administering of an injectable fluid (e.g., medication) to a patient, for example. The syringe barrel 12 is formed of a generally cylindrical wall 16 and an end member 18 that collectively define a chamber 20 for retaining fluid therein, with the syringe barrel 12 formed of glass or plastic, according to various embodiments. The syringe barrel 12 includes an open proximal end 22 configured to receive the plunger assembly 14 therein and a distal end 24 at which end member 18 is positioned. The proximal end 22 of the syringe barrel 12 may include a flange 26 to facilitate handling and positioning of the syringe 10 and to maintain the relative position of the syringe barrel 12 with respect to the plunger assembly 14 during medication administration. At the distal end 24, the end member 18 may include a shoulder 28 which narrows with respect to the cylindrical outer wall 16, as well as a hub portion 30 extending out distally from shoulder 28. The hub portion 30 is formed as a partially hollow member that defines a channel 32 therethrough in fluid communication with the chamber 20. A needle 34 is attached to the hub portion 30 within channel 32, such as by being glued or otherwise secured to the hub portion 30. According to some aspects of the disclosure, syringe 10 may further include a cover 35 that couples to the hub portion 30 of syringe barrel 12 to protect the needle 34.

The plunger assembly 14 of syringe 10 is formed of an elongate plunger rod 36 and a plunger head or stopper 38. The plunger rod 36 may include a main body 40 extending between a plunger proximal end 42 and a plunger distal end 44. In some embodiments, the main body 40 may include a plurality of elongate vanes or walls 46 extending axially along a length thereof between the plunger proximal end 42 and the plunger distal end 44. A thumb press 48 is positioned at the plunger proximal end 42 that may be engaged by a thumb (or other finger) of the user to apply a distally directed force to the plunger assembly 14 to move the plunger rod 36 with respect to the syringe barrel 12. The plunger distal end 44 includes a distal end head 50 that may abut/engage a proximal end of the stopper 38, to provide for a distal advancement thereof within syringe barrel 12, as explained in further detail below. In some embodiments, the distal end head 50 may be integrally formed with the main body 40 of the plunger rod 36, such as by the plunger rod 36 being formed via an injection molding process. In other embodiments, the distal end head 50 may be provided as a separate component from the main body 40 of the plunger rod 36, with the distal end head 50 coupled to the main body 40 (e.g., via a snap fit or other mechanical fastening means) to form the plunger rod 36.

The stopper 38 of plunger assembly 14 is positioned at the plunger distal end 44 so as to be movable along with the plunger rod 36 within the chamber 20 of syringe barrel 12. The stopper 38 may be made from a material that is different from the material of the plunger rod 36 and that is capable of forming a tight seal with the syringe barrel 12 as it is advanced therethrough. In some embodiments, the stopper 38 is formed of an elastomeric material such as butyl rubber, styrene butadiene rubber, poly isoprene rubber, liquid silicone rubber, as non-limiting examples. In other embodiments, the stopper 38 may instead be formed of a polymeric material, such as cross-linked or thermoplastic elastomers, as non-limiting examples.

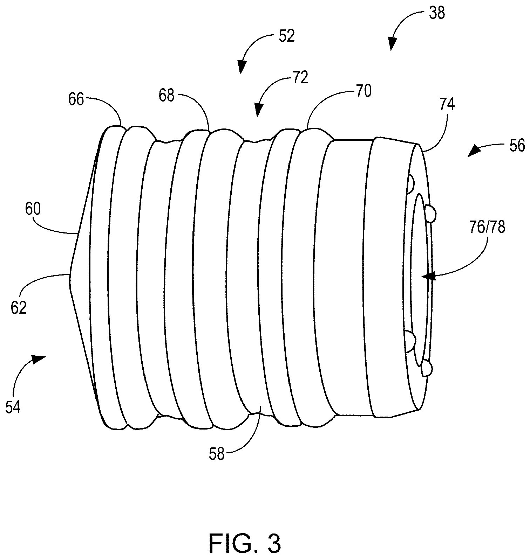

Referring now to FIG. 3, and with continued reference to FIGS. 1 and 2, the structure of stopper 38 is shown in greater detail, according to an aspect or embodiment of the disclosure. The stopper 38 is defined by a main body 52 that a closed distal end 54 configured to engage with the barrel 12 of syringe 10 and a proximal end 56 that abuts/engages with the distal end of plunger rod 36. In some embodiments, the proximal end 56 is configured as an open proximal end, but it is recognized that the proximal end 56 could instead be configured as a closed proximal end. The closed distal end 54 of the main body 52 includes a generally cylindrical portion 58 and a roof portion 60 that extends distally from cylindrical portion 58. In one embodiment, the roof portion 60 is configured as a conical portion that extends distally from cylindrical portion 58 to a tip 62 to provide the closed distal end 54. However, it is recognized that roof portion 60 may have other suitable shapes, such as being formed as a flat surface or domed surface, as other non-limiting examples.

An outer surface 64 of the main body 52 includes a plurality of ribs 66, 68, 70 formed thereon that extend circumferentially around the entire outer surface 64. In the illustrated embodiment, the plurality of ribs 66, 68, 70 includes a first rib 66, a second rib 68, and a third rib 70, although it is recognized that the stopper 38 could include a lesser or greater number of ribs, such as two ribs or four ribs. The ribs 66, 68, 70 are spaced apart longitudinally along the stopper 38, with an inter-rib region 72 present between each adjacent pair of ribs. According to embodiments of the disclosure, the structure of each of the ribs 66, 68, 70 may be controlled to achieve a desired interaction between the stopper 38 and the barrel 12, with the ribs 66, 68, 70 extending radially outward a desired distance from the outer surface 64 (i.e., a rib thickness) to act as bearing points against the inner surface of the syringe barrel 12.

In embodiments where the stopper 38 includes an open proximal end 56, the stopper 38 includes an annular shoulder 74 that defines/forms the proximal end and that defines an opening 76. The opening 76 provides access to an inner cavity 78 of the stopper. As known in the art, the inner cavity 78 may, in some embodiments, include a threaded inner surface, while in other embodiments the inner cavity 78 may be configured such that it does not include a thread/threads on the inner surface thereof.

According to aspects of the disclosure, the distal end head 50 of plunger rod 36 is configured to provide for a desired interaction with the syringe barrel 12 and the stopper 38 - with the extension member 50 specifically configured to enable a distal advancement of the stopper 38 within syringe barrel 12 during performing of an injection, while also being configured to enable a removal of the plunger rod 36 from the syringe barrel 12 after injection without requiring a disconnection from the stopper 38. According to embodiments of the disclosure, the distal end head 50 may be designed so as to enable separation of the plunger rod 36 from the stopper 38 and removal of the plunger rod 36 from the syringe barrel 12 responsive to application of a (uniaxial) traction force or motion to the plunger rod in a proximal direction.

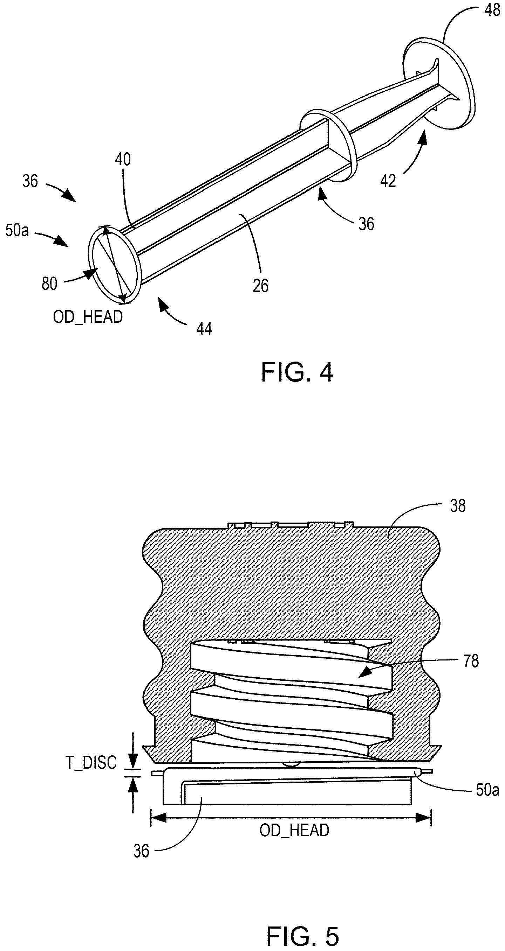

Referring to FIGS. 4-5, an embodiment of plunger rod 36 is shown according to one aspect of the disclosure. The plunger rod 36 is structured as previously described, with the plunger rod 36 generally including a main body 40 (with elongate vanes or walls 46) extending between a plunger proximal end 42 and a plunger distal end 44, with a thumb press 48 provided at the plunger proximal end 42 and a distal end head 50 provided at the plunger distal end 44.

As shown in FIGS. 4 and 5, the distal end head 50 is constructed as a circular or disc-shaped end member 50a that is constructed similar to thumb press 48. The disc-shaped end member 50a includes a distal-facing surface 80 that is generally flat or planar, that enables the end member to make flush contact with the stopper 38. The disc-shaped end member 50a has a thickness, T_disc, that is small enough to provide some flexibility threreto even when the disc-shaped end member 50a is formed of a generally rigid or semi-rigid polymeric material (e.g., a moldable polymeric material). In some non-limiting embodiments, the thickness, T disc, of the disc-shaped end member 50a may be between 0.01 and 0.5 mm.

According to aspects of the disclosure, the disc-shaped end member 50a is constructed to have an outer diameter, OD_head, that is greater than an inner diameter of the cavity 78 of stopper 38, ID_cavity. With the outer diameter of the disc-shaped end member 50a, OD_head, being greater than the inner diameter of the stopper cavity 78, ID_cavity, the distal-facing surface 80 of disc-shaped end member 50a abuts the annular shoulder 74 when the plunger rod 36 is brought into contact with the stopper 38, such that the disc-shaped end member 50a can apply a distally-directed force to the stopper 38 when the plunger rod 36 it pressed by a user, and thereby advance the stopper 38 distally within the syringe barrel 12.

The disc-shaped end member 50a is also constructed such that the outer diameter thereof, OD_head, is approximately equal to an inner diameter of the syringe barrel 12, ID_barrel (e.g., within 0 to +0.5 mm). With the outer diameter of the disc-shaped end member 50a, OD_head, being approximately equal to an inner diameter of the syringe barrel 12, ID_barrel, the disc-shaped end member 50a is in contact with an inner surface of the syringe barrel 12, so as to help maintain a straight/parallel alignment of the plunger rod 36 within syringe barrel 12 as the plunger rod 36 is advanced distally therein. The disc-shaped end member 50a may thus apply an evenly dispersed, distally-directed force to the stopper 38 when advancing the stopper 38 within the syringe barrel 12, thereby prevented tipping of the stopper 38. Additionally, the thin, flexible construction of the disc-shaped end member 50a allows for the disc-shaped end member 50a to maintain contact with the syringe barrel 12 as the plunger assembly 14 is advanced, with the flexibility of the disc-shaped end member 50a also helping to control/lower a gliding force needed to advance the plunger assembly 14 during injection.

With the plunger distal end 44 constructed to include a disc-shaped end member 50a as described above, the plunger rod 36 may be easily removed and separated from stopper 38 and syringe barrel 12 subsequent to completion of an injection. That is, as the disc-shaped end member 50a is not mechanically connected to the stopper 38, the plunger rod 36 can be removed from syringe barrel 12 via only an application of a (uniaxial) traction force or motion to the plunger rod 36 in the proximal direction.

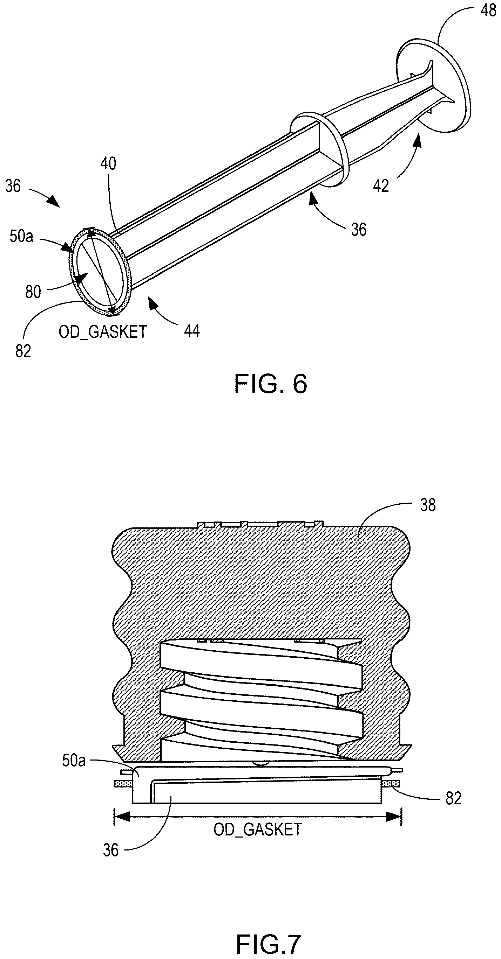

Referring to FIGS. 6-7, an embodiment of plunger rod 36 is shown according to another aspect of the disclosure. The plunger rod 36 is structured as previously described, with the plunger rod 36 generally including a main body 40 (with elongate vanes or walls 46) extending between a plunger proximal end 42 and a plunger distal end 44, with a thumb press 48 provided at the plunger proximal end 42 and a distal end head 50 provided at the plunger distal end 44.

As shown in FIGS. 6 and 7, the distal end head 50 is constructed similar to the distal end head 50 illustrated in FIGS. 4 and 5 - with the distal end head 50 including a disc-shaped end member 50a. However, in the embodiment of FIGS. 6 and 7, a gasket 82 is positioned about the main body 40 at the plunger distal end 44, with the gasket 82 positioned adjacent disc-shaped end member 50a (e.g., proximal to the disc-shaped end member 50a). The gasket 82 may be formed of an elastomeric material such as butyl rubber, styrene butadiene rubber, poly isoprene rubber, liquid silicone rubber, as non-limiting examples.

According to aspects of the disclosure, with the gasket 82 positioned about the main body 40 of plunger rod 36, the gasket 82 has an outer diameter, OD_gasket, that is equal to or slightly greater than an inner diameter of the syringe barrel 12, ID_barrel, such that an interference (e.g., 0 to 0.5 mm of interference) is provided between the gasket 82 and the syringe barrel 12. The interference between the gasket 82 and the syringe barrel 12 maintains the gasket 82 in contact with the syringe barrel 12 as the plunger assembly 14 is advanced and prevents the plunger rod 36 from falling out of the syringe barrel 12.

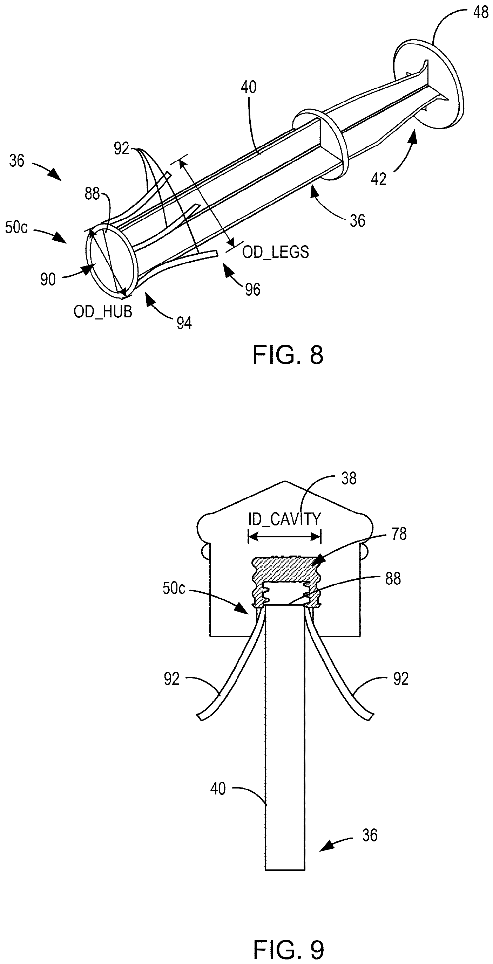

Referring to FIGS. 8-9, an embodiment of plunger rod 36 is shown according to another aspect of the disclosure. The plunger rod 36 is structured as previously described, with the plunger rod 36 generally including a main body 40 (with elongate vanes or walls 46) extending between a plunger proximal end 42 and a plunger distal end 44, with a thumb press 48 provided at the plunger proximal end 42 and a distal end head 50 provided at the plunger distal end 44.

As shown in FIGS. 8 and 9, the distal end head 50 is constructed as a multi-legged end member 50c. The multi-legged end member 50c comprises a central hub 88 having a flat distal-facing surface 90, along with a plurality of flexible legs 92 joined to the central hub 88 and spaced circumferentially thereabout. In some embodiments, the multi-legged end member 50c may include an arrangement of four circumferentially spaced-apart legs 92, although it is recognized that a greater or lesser number of legs 92 could be provided on multi-legged end member 50c - e.g., between two and six legs 92. Each of the flexible legs 92 comprises a first end 94 that is joined to the central hub 88 and a free second end 96, with the flexible legs 92 having a thin, elongated profile that allows the legs 92 to bend/flex responsive to a pressure applied thereto. The flexible legs 92 are configured to extend back proximally from the central hub 88 and flare radially outward therefrom. In some embodiments, each of the legs 92 comprises a curved or arc-shaped leg.

According to aspects of the disclosure, the central hub 88 of multi-legged end member 50c is constructed to have an outer diameter, OD_hub, that is less than an inner diameter of the cavity 78 of stopper 38, ID_cavity. With the outer diameter of the central hub 88, OD_hub, being less than the inner diameter of the stopper cavity 78, ID_cavity, the central hub 88 of plunger rod 36 may be inserted into the cavity 78 when the plunger rod 36 is brought into contact with the stopper 38, such that the central hub 88 can apply a distally-directed force to the stopper 38 when the plunger rod 36 it pressed by a user, and thereby advance the stopper 38 distally within the syringe barrel 12.

The legs 92 of the multi-legged end member 50c are flared outward such that the circumferential arrangement of legs 92 (at the second ends 96 thereof) has an outer diameter, OD_legs, that is approximately equal to an inner diameter of the syringe barrel 12, ID_barrel. With the outer diameter of the leg arrangement, OD_legs, being approximately equal to an inner diameter of the syringe barrel 12, ID _barrel, e.g., within 0 - 0.5 mm of interference, the multi-legged end member 50c (i.e., the second end 96 of legs 92) may keep contact with the syringe barrel 12 as the plunger rod 36 is advanced distally therein, so as to maintain a straight/parallel alignment of the plunger rod 36 within syringe barrel 12. As indicated above, the plurality of legs 92 are flexible in nature and thus, when the central hub 88 of plunger rod 36 is pushed into the cavity 78 of stopper 38, the legs 92 may flex/bend to enable such insertion, with the flexibility of the legs 92 also helping to control/lower a gliding force needed to advance the plunger assembly 14 during injection.

With the plunger distal end 44 constructed to include a multi-legged end member 50c as described above, the plunger rod 36 may be easily removed and separated from stopper 38 and syringe barrel 12 subsequent to completion of an injection. That is, as the multi-legged end member 50c is not mechanically connected or coupled to the stopper 38, the plunger rod 36 can be removed from syringe barrel 12 via only an application of a (uniaxial) traction force or motion to the plunger rod 36 in the proximal direction.

Beneficially, embodiments of the invention thus are directed to a plunger assembly 14 for a syringe having a plunger rod configured to enable an easy separation and removal of the plunger rod. The plunger rod may include a distal end head designed to enable advancement of a stopper within the syringe barrel without being mechanically coupled to the stopper, so that the plunger rod may be separated away from the stopper and removed from the syringe barrel responsive only to application of a proximally-directed traction force or motion to the plunger rod.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

## Claims

1. A syringe comprising:
a syringe barrel having a barrel proximal end and a barrel distal end and defining a chamber configured for containing a fluid therein; and
a plunger assembly axially movable within the chamber of the syringe barrel from a retracted position to an advanced position, the plunger assembly including:
a plunger rod having a plunger rod proximal end and a plunger rod distal end, the plunger rod comprising a distal end head positioned at the plunger rod distal end; and
a stopper secured to the plunger rod distal end, the stopper having a proximal end and a distal end;
wherein, with the plunger rod and stopper of the plunger assembly assembled, the distal end head is configured to abut the stopper without being mechanically coupled thereto, so that the plunger rod may be separated from the stopper and removed from the syringe barrel by applying only a rearward axial force on the plunger rod.

2. The syringe of claim 1, wherein the stopper comprises an inner cavity formed therein at the proximal end, with an annular shoulder at the proximal end of the stopper defining an opening to the inner cavity.

3. The syringe of claim 2, wherein the distal end head comprises a disk-shaped end member having a flat distal-facing surface.

4. The syringe of claim 3, wherein the disk-shaped end member has an outer diameter that is greater than an inner diameter of the cavity and approximately equal to an inner diameter of the syringe barrel, with the disk-shaped end member thus in contact with an inner surface of the syringe barrel.

5. The syringe of claim 4, wherein with the plunger rod and stopper of the plunger assembly assembled, the disk-shaped end member abuts the annular shoulder of the stopper.

6. The syringe of claim 4, wherein the disk-shaped end member has a thickness that provides for deforming of the disk-shaped end member as the plunger assembly is advanced distally into the syringe barrel during an injection.

7. The syringe of claim 3, wherein the thickness of the disk-shaped end member is between 0.01 and 0.5 mm.

8. The syringe of claim 6, further comprising a gasket positioned about the plunger rod adjacent the disk-shaped end member.

9. The syringe of claim 8, wherein the is gasket is formed of an elastomeric material.

10. The syringe of claim 8, wherein with the gasket positioned about the plunger rod, the gasket the has an outer diameter that is approximately equal to an inner diameter of the syringe barrel, with the gasket thus in contact with an inner surface of the syringe barrel.

11. The syringe of claim 10, wherein the gasket is in interference with the inner surface of the syringe barrel by 0 to 0.5 mm.

12. The syringe of claim 2, wherein the distal end head comprises a multi-legged end member, the multi-legged end member including:
a central hub having a flat distal-facing surface; and
a plurality of flexible legs joined to the central hub and spaced circumferentially thereabout, with each of the plurality of flexible legs comprising a first end that is joined to the central hub and a free second end, with each of the plurality of flexible legs having a thin, elongated profile that allows the leg to bend and flex responsive to a pressure applied thereto;
wherein the plurality of flexible legs are configured to extend back proximally from the central hub and flare radially outward therefrom.

13. The syringe of claim 12, wherein each of the plurality of flexible legs comprises a curved or arc-shaped leg.

14. The syringe of claim 12, wherein the central hub has an outer diameter, that is less than an inner diameter of the cavity such that, with the plunger rod and stopper of the plunger assembly assembled, the central hub is inserted into the cavity.

15. The syringe of claim 14, wherein a circumferential arrangement of the plurality of flexible legs, at the second ends thereof, has an outer diameter that is approximately equal to an inner diameter of the syringe barrel, with the second ends of the plurality of flexible legs thus in contact with an inner surface of the syringe barrel.

16. The syringe of claim 14, wherein with the plunger rod and stopper of the plunger assembly assembled and the central hub inserted into the cavity, the plurality of flexible legs bend to enable the insertion.

17. The syringe of claim 14, wherein the plurality of flexible legs comprises four legs.

18. The syringe of claim 1, wherein the distal end head maintains contact with an inner surface of the syringe barrel, without increasing a gliding force of the plunger assembly during a distal advancement thereof within the syringe barrel.

19. The syringe of claim 1, wherein the distal end head is formed integrally with a main body of the plunger rod.

20. The syringe of claim 1, wherein the distal end head is coupled to a main body of the plunger rod.

21. The syringe of claim 1, wherein the syringe comprises a pre-filled syringe.
